# EUROPÄISCHE PATENTANMELDUNG

(11) **EP 3 324 469 A1**
(43) Veröffentlichungstag der Anmeldung: **23.05.2018**
(21) Anmeldenummer: 16199561.8
(22) Anmeldetag: 18.11.2016
(51) Int. Cl.: H01M 4/72, H01M 4/74, A61N 1/372

(54) **ABLEITUNGSGITTER FÜR EINE ELEKTROCHEMISCHE SPANNUNGSQUELLE**

(71) Anmelder: LITRONIK Batterietechnologie GmbH, 01796 Pirna (DE)
(72) Erfinder: Kaiser, Annett, 01809 Heidenau (DE); Rodig, Thomas, 01796 Pirna (DE)
(74) Vertreter: Galander, Marcus

(57) **Zusammenfassung**

Die Erfindung betrifft ein Ableitungsgitter (1) für eine Kathode einer elektrochemischen Spannungsquelle, wobei das Ableitungsgitter (1) sich entlang einer Erstreckungsebene erstreckt und einen äußeren umlaufenden Randbereich (2) aufweist. Erfindungsgemäß ist vorgesehen, dass von dem Randbereich (2) eine Mehrzahl an Positionsnasen (20) nach außen hin in der Erstreckungsebene des Ableitungsgitters (1) abstehen.

## Beschreibung

Die vorliegende Erfindung betrifft ein Ableitungsgitter für eine Kathode einer elektrochemischen Spannungsquelle gemäß Anspruch 1.

Derartige Ableitungsgitter dienen zum Tragen eines aktiven Kathodenmaterials (z. B. geeignete Si- oder Mn-Verbindungen), das auf das Ableitungsgitter aufgebracht wird.

Aus dem Stand der Technik sind derartige Ableitungsgitter bekannt. So beschreibt z. B. die EP 2 056 381 B1 ein derartiges Ableitungsgitter, das Vorsprünge normal zur Gitterebene aufweist, die ein Anhaften des aktiven Kathodenmaterials verbessern sollen.

Weiterhin ist aus der US 2005/0064291 A1 ein Ableitungsgitter bekannt, das ebenfalls derartige Vorsprünge aufweist, mit dem Ziel einer Erhöhung der Energiedichte.

Grundsätzlich besteht bei den bisher verwendeten Ableitungsgittern die Problematik, dass es im Randbereich der Kathoden zu Abhebungen bzw. Abplatzungen von Kathodenmaterial sowie zu Rissen im Kathodenmaterial kommen kann, was auf eine unzureichende Haftung des Kathodenmaterials am Ableitungsgitter oder auf eine zu geringe Einbettung bzw. Umhüllung des Gitters mit Kathodenmaterial zurückgeführt werden kann.

Ein derartiges Ablösen des aktiven Kathodenmaterials ist insbesondere bei Kathodenmaterialien mit kleinen Partikelgrößen, wie z. B. CFx oder Braunsteingranulat zu beobachten.

Hiervon ausgehend liegt der vorliegenden Erfindung die Aufgabe zugrunde, ein Ableitungsgitter zu schaffen, das im Hinblick auf die oben genannte Problematik verbessert ist.

Diese Aufgabe wird durch ein Ableitungsgitter mit den Merkmalen des Anspruchs 1 gelöst. Vorteilhafte Ausgestaltungen dieses Erfindungsaspekts sind in den Unteransprüchen angegeben und werden nachfolgend beschrieben.

Weitere Aspekte der Erfindung betreffen eine Kathode, ein Verfahren zur Herstellung einer Kathode, eine elektrochemische Spannungsquelle (hier auch als Batterie bezeichnet) sowie ein batteriebetriebenes Gerät.

Gemäß Anspruch 1 wird ein Ableitungsgitter für eine Kathode einer elektrochemischen Spannungsquelle offenbart, wobei das Ableitungsgitter sich entlang einer Erstreckungsebene erstreckt und einen äußeren umlaufenden Randbereich aufweist, und wobei erfindungsgemäß von dem Randbereich eine Mehrzahl an Positionsnasen nach außen hin in der Erstreckungsebene des Ableitungsgitters abstehen.

Durch die solchermaßen zurückgesetzte Außenkontur des Ableitungsgitters (zwischen den Positionsnasen) ist es nun möglich, dass sich das Kathodenmaterial beim Verpressen beiderseits des umlaufenden Randbereiches miteinander verbinden kann und somit den Randbereich im Querschnitt umgibt (zumindest abseits der Positionsnasen). Das Ableitungsgitter wird somit nunmehr von dem Kathodenmaterial fast vollständig umhüllt und in dieses eingebettet.

Hierdurch werden mit Vorteil Risse am umlaufenden Rand der Kathode sowie ein Abplatzen des Kathodenmaterials vom Ableitungsgitter verhindert und im Ergebnis eine umlaufend stabile und geschlossene Kathode hergestellt.

Gemäß einer bevorzugten Ausführungsform des erfindungsgemäßen Ableitungsgitters ist vorgesehen, dass die Positionsnasen dazu ausgebildet sind, in einem Presswerkzeug an einer umlaufenden Wandung des Presswerkzeugs anzuliegen, derart, dass sich das Ableitungsgitter im Presswerkzeug nicht mehr in einer beliebigen, in der Erstreckungsebene liegenden Richtung gegenüber dem Presswerkzeug bewegen kann. Somit wird in vorteilhafterweise eine definierte Positionierung des Ableitungsgitters in der Pressmatrize (Presswerkzeug) ermöglicht.

Weiterhin ist gemäß einer bevorzugten Ausführungsform des erfindungsgemäßen Ableitungsgitters vorgesehen, dass das Ableitungsgitter in der Erstreckungsebene an allen vier Seiten bzw. an den entsprechenden aneinander angrenzenden Abschnitten des umlaufenden Randbereiches jeweils zumindest eine Positionsnase aufweist.

Weiterhin ist gemäß einer bevorzugten Ausführungsform des erfindungsgemäßen Ableitungsgitters vorgesehen, dass der Randbereich eine wellenförmige Kontur mit alternierend angeordneten Bergen und Tälern aufweist, wobei die Berge die besagten Positionsnasen bilden.

In dieser Ausführungsform weist der Gitterrand also insbesondere mit anderen Worten eine Wellenform auf, so dass eine Vielzahl an Randabschnitten vorhanden ist, wobei sich an jedem Randabschnitt Kathodenmaterial der oberen und unteren Füllschicht miteinander verbinden kann.

Weiterhin ist gemäß einer bevorzugten Ausführungsform des erfindungsgemäßen Ableitungsgitters vorgesehen, dass die einzelnen Positionsnasen bzw. deren Kontur in der Draufsicht auf die Erstreckungsebene jeweils rechteckförmig, rautenförmig oder abgerundet ausgebildet sind. Andere Konturen sind ebenfalls denkbar.

Weiterhin ist gemäß einer bevorzugten Ausführungsform des erfindungsgemäßen Ableitungsgitters vorgesehen, dass das Ableitungsgitter eine Vielzahl an mit dem umlaufenden Randbereich (vorzugsweise einstückig bzw. integral) verbundenen Streben aufweist, die eine Gitterstruktur mit einer Vielzahl an Gitterzellen ausbilden.

Weiterhin ist gemäß einer bevorzugten Ausführungsform des erfindungsgemäßen Ableitungsgitters vorgesehen, dass das Ableitungsgitter dabei eine Vielzahl an hexagonalen Gitterzellen aufweist. Diese werden insbesondere durch integral (einstückig) miteinander verbundene Streben des Gitters gebildet. Hierbei kann das Ableitungsgitter auch Gitterzellen aufweisen (insbesondere am Randbereich), die von der hexagonalen Form abweichen.

Gemäß einer alternativen Ausführungsform ist vorgesehen, dass das Ableitungsgitter eine Vielzahl an rautenförmigen Gitterzellen aufweist, wobei insbesondere jede rautenförmige Gitterzelle durch vier einstückig miteinander verbundene Streben gebildet ist, wobei insbesondere von jeder dieser Streben zwei Vorsprünge in entgegengesetzten Richtungen in der Erstreckungsebene des Ableitungsgitters abstehen. Auch in dieser Ausführungsform kann das Ableitungsgitter Gitterzellen aufweisen (insbesondere am Randbereich), die von der Rautenform abweichen.

Weiterhin ist gemäß einer bevorzugten Ausführungsform der Erfindung vorgesehen, dass das Ableitungsgitter eine vom Randbereich in der Erstreckungsebene des Ableitungsgitters abstehende Kontaktfahne aufweist. Die Kontaktfahne ist insbesondere dazu vorgesehen, die Kathode am Kontaktstück, welches die elektrisch leitende Verbindung nach außen darstellt, zu fixieren und so die Kathode elektrisch zu kontaktieren.

Ein weiterer Aspekt der vorliegenden Erfindung betrifft eine Kathode für eine elektrochemische Spannungsquelle, wobei die Kathode ein erfindungsgemäßes Ableitungsgitter aufweist, wobei auf das Ableitungsgitter beidseitig ein Kathodenmaterial aufgebracht ist.

Bei dem Kathodenmaterial kann es sich insbesondere um eines der folgenden Materialien handeln: Mangandioxid MnO₂, Kupferoxiphosphate CxP, Kohlenstoffmonofluorid CFx und/oder deren Mischungen bzw. Zusatzstoffe wie Graphit, Ruß und Bindemittel

Weiterhin kann das Ableitungsgitter aus einem der folgenden Materialien bestehen: Edelstahl, Titan, Kupfer, Aluminium.

Gemäß einer bevorzugten Ausführungsform der erfindungsgemäßen Kathode ist vorgesehen, dass das Ableitungsgitter derart in das Kathodenmaterial eingebettet ist, dass der Randbereich des Ableitungsgitters zumindest abschnittweise, insbesondere zwischen je zwei benachbarten Positionsnasen, im Querschnitt vollständig von dem Kathodenmaterial umgeben ist (ggf. mit Ausnehme derjenigen Abschnitte des Randbereiches von denen Streben nach innen hin abgehen).

Weiterhin betrifft die Erfindung eine elektrochemische Spannungsquelle, die ein erfindungsgemäßes Ableitungsgitter oder eine erfindungsgemäße Kathode aufweist.

Weiterhin betrifft die Erfindung ein batteriebetriebenes Gerät mit einer elektrisch betriebenen Funktionseinheit und einer erfindungsgemäßen elektrochemischen Spannungsquelle zum Betreiben der Funktionseinheit.

Gemäß einer Ausführungsform der Erfindung ist das batteriebetriebene Gerät vorzugsweise dazu eingerichtet und vorgesehen, in einen menschlichen oder tierischen Körper implantiert zu werden.
Bevorzugt ist das batteriebetriebene Gerät eines der folgenden Geräte:
- ein Herzschrittmacher,
- ein Defibrillator,
- ein Neurostimulator, oder
- eine Medikamentenpumpe (z. B. für Insulin).

Schließlich betrifft ein weiterer Aspekt der vorliegenden Erfindung ein Verfahren zum Herstellen einer insbesondere erfindungsgemäßen Kathode, wobei ein erfindungsgemäßes Ableitungsgitter bereitgestellt wird und in ein Presswerkzeug eingelegt wird, so dass die Positionsnasen an einer umlaufenden Wandung des Presswerkzeugs anliegen und das Ableitungsgitter in seiner Erstreckungsebene bezüglich des Presswerkzeugs fixiert ist (d. h., dass Ableitungsgitter kann sich nicht mehr in einer beliebigen in der Erstreckungsebene liegenden Richtung gegenüber dem Presswerkzeug bewegen), wobei mittels des Presswerkzeugs ein vorzugsweise pulverförmiges Kathodenmaterial beidseitig auf das Ableitungsgitter aufgepresst wird, insbesondere so, dass der umlaufende Randbereich des Ableitungsgitters zumindest abschnittsweise im Querschnitt vollständig von dem Kathodenmaterial umgeben ist (siehe auch oben).

Die Erfindung ermöglicht zusammenfassend eine umlaufend geschlossene Kontur der Kathode, verbunden mit einer Verbesserung der mechanischen Eigenschaften hinsichtlich der Stabilität der Kathode im Randbereich.

Weitere Merkmale und Vorteile der Erfindung sollen nachfolgend anhand von Figurenbeschreibungen von Ausführungsformen der Erfindung beschrieben werden. Es zeigen:
- Fig. 1: eine Draufsicht auf ein erfindungsgemäßes Ableitungsgitter;
- Fig. 2: eine Draufsicht auf ein weiteres erfindungsgemäßes Ableitungsgitter;
- Fig. 3: eine Draufsicht auf ein weiteres erfindungsgemäßes Ableitungsgitter; und
- Fig. 4: eine Draufsicht auf ein in einem Presswerkzeug angeordnetes Ableitungsgitter gemäß Figur 1.

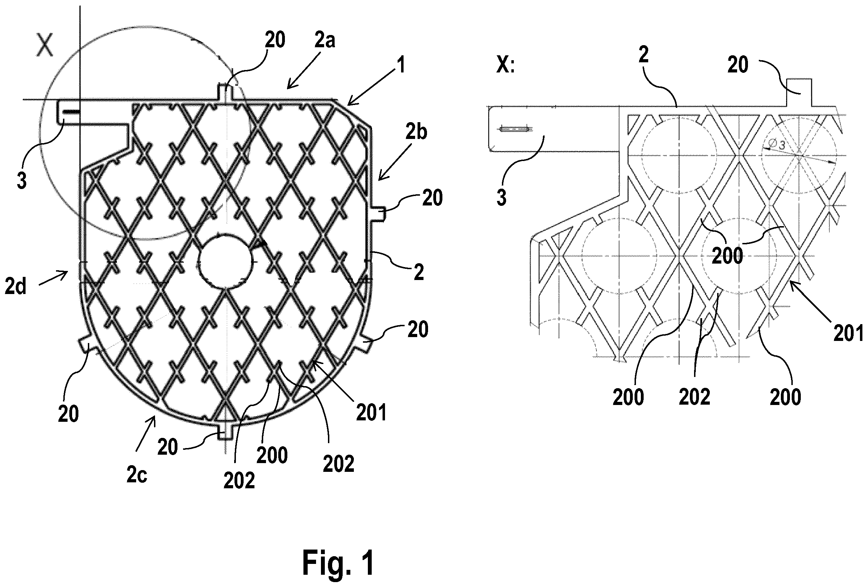

Figur 1 zeigt ein Ableitungsgitter 1 für eine Kathode einer elektrochemischen Spannungsquelle. Zur Herstellung der eigentlichen Kathode wird ein aktives Kathodenmaterial, z. B. MnO₂, mit dem Ableitungsgitter 1 in einem Presswerkzeug insbesondere kalt verpresst, so dass das Ableitungsgitter 1 in das Kathodenmaterial eingebettet wird. Zur Herstellung einer elektrochemischen Spannungsquelle werden beidseitig der Kathode (insbesondere kongruente) Separatorenlagen (z. B. aus PE/PP) angeordnet, auf die wiederum je eine (insbesondere kongruente) Anode (z. B. Li-Anode) angeordnet wird. Dieser Elektrodenstapel wird in einem Gehäuse angeordnet und das Gehäuse mit einem geeignete Elektrolyten (z. B. ein Elektrolytsystem für MnO₂) gefüllt und sodann hermetisch verschlossen. Eine Kontaktfahne 3 der Kathode sowie eine Kontaktfahne für die Anoden werden aus dem Gehäuse herausgeführt, so dass die Elektroden von außen kontaktierbar sind.

Im Einzelnen erstreckt sich das Ableitungsgitter gemäß Figur 1 flach in einer Erstreckungsebene und weist dabei einen äußeren umlaufenden Randbereich 2 auf. Von dem Randbereich gehen nach innen hin in der Erstreckungsebene eine Vielzahl an Streben 200 ab, die einstückig mit dem Randbereich verbunden sind. Die Streben 200 des Ableitungsgitters bilden eine Gitterstruktur mit einer Mehrzahl an Gitterzellen aus, wobei das in der Figur 1 gezeigte Ableitungsgitter 1 eine Mehrzahl an miteinander verbundenen rautenförmigen Gitterzellen 201 aufweist, wobei bevorzugt jede rautenförmige Gitterzelle 201 durch vier miteinander verbundene sowie rautenförmig zueinander angeordnete Streben 200 gebildet ist, wobei insbesondere von jeder dieser Streben 200 mittig zwei Vorsprünge 202 in entgegengesetzten Richtungen in der Erstreckungsebene des Ableitungsgitters 1 abstehen.

Weiterhin weist das Ableitungsgitter 1 eine vom Randbereich 2 in der Erstreckungsebene des Ableitungsgitters 1 abstehende Kontaktfahne 3 auf, die insbesondere parallel zu einem ersten Randabschnitt 2a des Ableitungsgitters 1 erstreckt ist.

Erfindungsgemäß gehen vom Randbereich 2 des Ableitungsgitters eine Mehrzahl an Positionsnasen 20 nach außen hin in der Erstreckungsebene des Ableitungsgitters 1 ab, die hier rechteckförmig ausgebildet sind.

Die Positionsnasen 20 dienen insbesondere zum Zentrieren des Ableitungsgitters 1 in einer Matrize M des Presswerkzeugs und bewirken dabei, dass das aktive Kathodenmaterial auch außen am Randbereich 2 angeordnet wird, so dass der Randbereich zumindest abschnittsweise in umfänglicher Richtung des Gitters 1 vollständig mit dem Kathodenmaterial umhüllt ist. Dies bedingt im Randbereich 2 eine bessere Anhaftung des Kathodenmaterials.

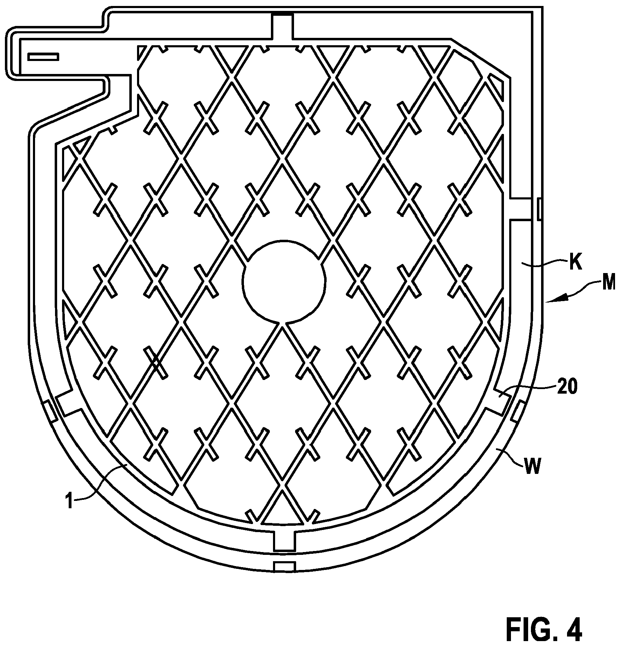

Die Zentrierungsfunktion ist in der Figur 4 gezeigt, die eine Ansicht von oben in die Matrize bzw. das Presswerkzeug M zeigt. Die Positionsnasen fixieren das Gitter 1 in dem Presswerkzeug, indem sie von innen an der umlaufenden Wandung W des Presswerkzeuges M anliegen, so dass das Gitter 1 zentriert in das Kathodenmaterial K eingebettet werden kann.

Bevorzugt sind die Positionsnasen 20 an allen vier Seiten bzw. Randabschnitten 2a 2b, 2c, 2d angeordnet und stehen dort jeweils in der Erstreckungsebene des Ableitungsgitters vom Randbereich nach außen hin ab.

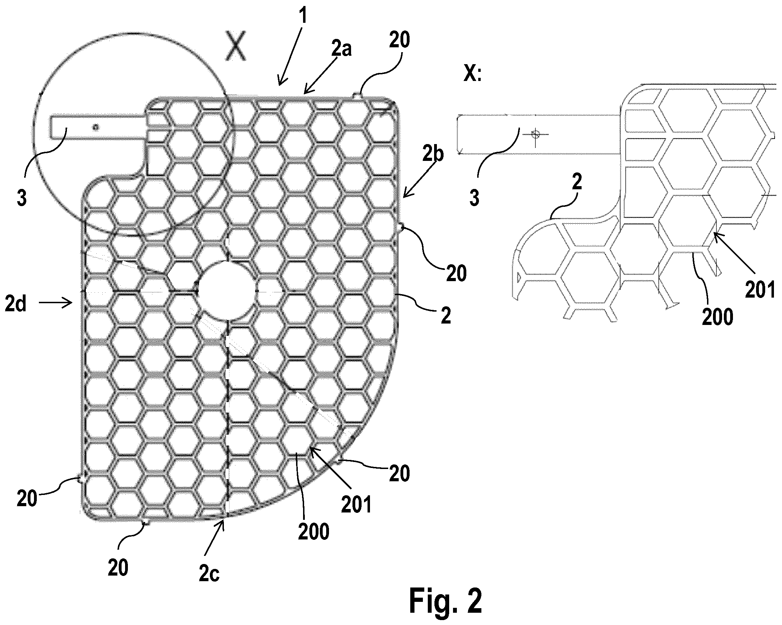

Figur 2 zeigt eine Abwandlung des in der Figur 1 gezeigten Ableitungsgitters 1, wobei hier im Unterschied zur Figur 1 die einzelnen Gitterzellen 201 bevorzugt hexagonal ausgebildet sind.

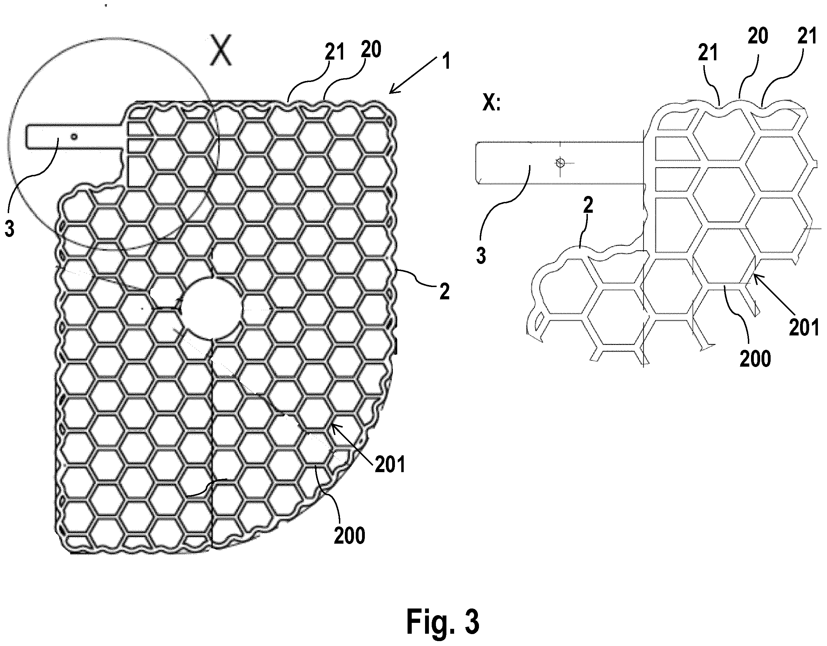

Schließlich zeigt die Fig. 3 eine Abwandlung des in der Figur 2 gezeigten Ableitungsgitters 1, wobei hier im Unterschied zur Figur 2 der Randbereich 2 des Ableitungsgitters 1 eine wellenförmige Kontur mit alternierend angeordneten Bergen 20 und Tälern 21 aufweist, wobei die Berge 20 die besagten Positionsnasen 20 zum Zentrieren des Ableitungsgitters 1 im Presswerkzeug bilden.

## Patentansprüche

1. Ableitungsgitter (1) für eine Kathode einer elektrochemischen Spannungsquelle, wobei das Ableitungsgitter (1) sich entlang einer Erstreckungsebene erstreckt und einen äußeren umlaufenden Randbereich (2) aufweist,
**dadurch gekennzeichnet,**
**dass** von dem Randbereich (2) eine Mehrzahl an Positionsnasen (20) nach außen hin in der Erstreckungsebene des Ableitungsgitters (1) abstehen.

2. Ableitungsgitter nach Anspruch 1, **dadurch gekennzeichnet, dass** die Positionsnasen (20) dazu ausgebildet sind, in einem Presswerkzeug an einer umlaufenden Wandung des Presswerkzeugs anzuliegen, derart, dass sich das Ableitungsgitter (1) im Presswerkzeug nicht mehr in einer beliebigen, in der Erstreckungsebene liegenden Richtung gegenüber dem Presswerkzeug bewegen kann.

3. Ableitungsgitter nach Anspruch 1 oder 2, **dadurch gekennzeichnet, dass** das Ableitungsgitter (1) in der Erstreckungsebene vier Seiten (2a, 2b, 2c, 2d) aufweist, wobei an jeder Seite zumindest eine Positionsnase (20) vorgesehen ist.

4. Ableitungsgitter nach einem der vorhergehenden Ansprüche, **dadurch gekennzeichnet, dass** der Randbereich (2) eine wellenförmige Kontur mit alternierend angeordneten Bergen (20) und Tälern (21) aufweist, wobei die Berge (20) die besagten Positionsnasen (20) bilden.

5. Ableitungsgitter nach einem der Ansprüche 1 bis 3, **dadurch gekennzeichnet, dass** die Positionsnasen (20) rechteckförmig, rautenförmig oder abgerundet ausgebildet sind.

6. Ableitungsgitter nach einem der vorhergehenden Ansprüche, **dadurch gekennzeichnet, dass** das Ableitungsgitter (1) eine Vielzahl an mit dem umlaufenden Randbereich (2) verbundenen Streben (200) aufweist, die eine Gitterstruktur mit einer Vielzahl an Gitterzellen (201) ausbilden.

7. Ableitungsgitter nach einem der vorhergehenden Ansprüche, **dadurch gekennzeichnet, dass** die Gitterzellen (201) hexagonal ausgebildet sind.

8. Ableitungsgitter nach einem der Ansprüche 1 bis 6, **dadurch gekennzeichnet, dass** die Gitterzellen (201) rautenförmig ausgebildet sind, wobei insbesondere jede rautenförmige Gitterzelle (201) durch vier miteinander verbundene Streben (200) gebildet ist, wobei insbesondere von jeder dieser Streben (200) zwei Vorsprünge (202) in entgegengesetzten Richtungen in der Erstreckungsebene des Ableitungsgitters (1) abstehen.

9. Ableitungsgitter nach einem der vorhergehenden Ansprüche, **dadurch gekennzeichnet, dass** das Ableitungsgitter (1) eine vom Randbereich (2) in der Erstreckungsebene des Ableitungsgitters (1) abstehende Kontaktfahne (3) aufweist.

10. Kathode für eine elektrochemische Spannungsquelle, wobei die Kathode ein Ableitungsgitter (1) nach einem der vorhergehenden Ansprüche aufweist, wobei auf das Ableitungsgitter (1) beidseitig ein Kathodenmaterial (K) aufgebracht ist.

11. Kathode nach Anspruch 10, **dadurch gekennzeichnet, dass** das Ableitungsgitter (1) derart in das Kathodenmaterial eingebettet ist, dass der umlaufende Randbereich (2) des Ableitungsgitters (1) zumindest abschnittsweise im Querschnitt vollständig von dem Kathodenmaterial umgeben ist.

12. Elektrochemische Spannungsquelle mit einem Ableitungsgitter (1) nach einem der Ansprüche 1 bis 9 oder mit einer Kathode nach einem der Ansprüche 10 oder 11.

13. Batteriebetriebenes Gerät mit einer elektrisch betriebenen Funktionseinheit und einer elektrochemischen Spannungsquelle zum Betreiben der Funktionseinheit gemäß Anspruch 12.

14. Batteriebetriebenes Gerät nach Anspruch 13, **dadurch gekennzeichnet, dass** das batteriebetriebene Gerät in einen menschlichen oder tierischen Körper implantierbar ist, wobei insbesondere das batteriebetriebene Gerät eines der folgenden Geräte ist: ein Herzschrittmacher, ein Defibrillator, ein Neurostimulator, eine Medikamentenpumpe.

15. Verfahren zum Herstellen einer Kathode, insbesondere nach einem der Ansprüche 10 oder 11, wobei ein Ableitungsgitter (1) gemäß einem der Ansprüche 1 bis 9 bereitgestellt wird und in ein Presswerkzeug eingelegt wird, so dass die Positionsnasen (20) an einer umlaufenden Wandung (W) des Presswerkzeugs (M) anliegen und das Ableitungsgitter (1) in seiner Erstreckungsebene bezüglich des Presswerkzeugs fixiert ist, wobei mittels des Presswerkzeugs Kathodenmaterial (K) beidseitig auf das Ableitungsgitter (1) aufgepresst wird, insbesondere so, dass der Randbereich (2) des Ableitungsgitters (1) zumindest abschnittsweise im Querschnitt vollständig von dem Kathodenmaterial (K) umgeben ist.
